# FASCICULE DE BREVET EUROPEEN

(11) **EP 4 585 243 B1**
(45) Date de publication et mention de la délivrance du brevet: **12.08.2026**
(21) Numéro de dépôt: 24216273.3
(22) Date de dépôt: 28.11.2024
(51) Int. Cl.: A61M 16/00, A61M 16/06, A61M 16/10, A61M 16/12, A61M 16/20, A61M 16/22, A61M 16/08, B01D 53/00, B01D 53/22

(54) **INSTALLATION DE FOURNITURE DE GAZ RESPIRATOIRE EN CIRCUIT FERMÉ COMPRENANT UN MODULE DE PERMÉATION**
ATEMGASVERSORGUNGSANLAGE MIT GESCHLOSSENEM KREISLAUF UND EINEM PERMEATIONSMODUL
CLOSED-CIRCUIT BREATHING GAS SUPPLY INSTALLATION COMPRISING A PERMEATION MODULE

(30) Priorité: 15.01.2024 FR 2400343
(43) Date de publication de la demande: 16.07.2025
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75007 Paris (FR)
(74) Mandataire: Air Liquide

(56) Documents cités:
- EP-A1- 4 108 282
- US-A- 3 489 144
- US-A1- 2007 017 516
- US-A1- 2007 151 561
- US-A1- 2020 297 964

## Description

La présente invention concerne une installation de fourniture de gaz respiratoire, fonctionnant en circuit fermé, incluant un système de purification et un système de recyclage des gaz expirés par le patient permettant une fourniture d'un mélange gazeux enrichi en oxygène (O₂) au patient en minimisant la consommation d'oxygène provenant d'une source d'oxygène, telle une bouteille d'oxygène sous pression, alimentant l'installation.

L'administration d'O₂ gazeux permet de corriger une situation hypoxémique chez une personne humaine, i.e. un patient, quel que soit son âge, i.e. nouveaux nés, enfants, adolescents, adultes, personnes âgées ou autres, souffrant une pathologie respiratoire engendrant une saturation d'oxyhémoglobine, mesurée dans le sang, inférieure à une valeur de référence reflétant une saturation « normale », par exemple une saturation inférieure à 90%, par exemple une pathologie respiratoire du type Bronchopneumopathie Chronique Obstructive (BPCO), Syndrome de Détresse Respiratoire Aigüe (SDRA) ou autre.

L'oxygène de qualité médicale est fourni au patient « hypoxique » soit au sein d'un l'hôpital, soit hors hôpital, par exemple dans une unité de secours mobile (SAMU, ambulances...) ou encore au domicile du patient considéré.

Selon le cas, l'oxygène peut provenir soit d'une canalisation de gaz, typiquement d'un réseau de fluides hospitalier, soit d'une bouteille de gaz sous pression contenant par exemple de 400 à 1000 L d'O₂ (vol. gazeux) comprimé jusqu'à 200 bar abs ou plus (mesuré à 1 atm).

Le débit d'oxygène délivré par une bouteille de gaz est réglé selon l'indication visée. Dans les cas les plus aigus, il peut être d'environ 15 L/min pour garantir une haute concentration d'oxygène inhalé, c'est-à-dire proche de 100%, comme par exemple en situation d'urgence lors d'une noyade, trauma majeur, choc septique, intoxication au monoxyde de carbone (CO) ou bien de détresse respiratoire aigüe (due par exemple à la COVID-19).

Dans tous les cas, le but de l'administration d'oxygène, quel que soit le débit, est de permettre à la saturation en oxyhémoglobine de revenir à une valeur normale, typiquement d'au moins 90% environ.

Pour des raisons d'encombrement et de poids, beaucoup d'unités d'urgence, tels que pompiers, de sauveteurs ou militaires, préfèrent utiliser une source d'oxygène gazeux aussi compacte que possible, par exemple une bouteille de 2L de volume interne (équiv. en eau) remplie d'oxygène (O₂) comprimé à 200 bar, soit un volume de l'ordre de 400 L d'oxygène (pleine).

Or, on comprend que l'autonomie conférée par une telle bouteille, c'est-à-dire le temps d'utilisation avant qu'elle ne soit vide de gaz, varie en fonction du débit de soutirage. Ainsi, elle est inférieure à 30 min pour un débit de 15 L/min.

L'autonomie peut dès lors s'avérer insuffisante, par exemple lors d'un transport de patient de longue durée, c'est-à-dire de plusieurs heures, typiquement lors d'une évacuation sanitaire ou depuis une zone reculée, comme lors d'une opération militaire sur théâtre d'opération extérieur, ou pendant un secours aux victimes en cas de désastre majeur, tel un tremblement de terre ou autre.

US-A-2021/0121649 propose un système de fourniture de gaz, i.e. d'oxygène, en circuit fermé composé d'une bouteille d'oxygène comprimé munie d'un détendeur de gaz, d'une tubulure alimentant un réservoir de gaz et un module contenant de la chaux sodée pour purifier les gaz expirés par le patient (i.e. victime) en piégeant le dioxyde de carbone (CO₂) qu'y s'y trouve. Il réinhale ensuite ses propres gaz expirés débarrassés du CO₂. Sachant que près de 95% de l'oxygène inhalé est expiré (i.e. non métabolisé), purifier les gaz expirés permet de considérablement réduire le débit fourni par la source d'oxygène, donc d'augmenter l'autonomie puisque le débit d'oxygène métabolisé est inférieur à 1L/min environ.

Cependant, un tel système n'est pas idéal car il ne permet qu'une utilisation limitée dans le temps. En effet, le piégeage du CO₂ dans le module contenant de la chaux sodée repose sur une réaction chimique au cours de laquelle le CO₂ réagit avec la chaux sodée pour former du carbonate de calcium. Ainsi, en fonction de la quantité de chaux sodée disponible dans le module, les sites de réaction vont diminuer progressivement jusqu'à ce que plus aucune réaction ne soit possible. Le module est alors saturé et le CO₂ ne peut plus être capté. Afin d'obtenir une autonomie acceptable en terme de piégeage de CO₂, une plus grande quantité de chaux sodée peut être pourvue mais ceci va à l'encontre des besoins de compacité, sans évoquer par ailleurs la gestion des déchets, car le carbonate de calcium ne peut être recyclé.

D'autres installations de fourniture de gaz comprenant notamment des modules de purification de type tamis moléculaire sont enseignées par les documents EP-A-4108282, US-A-2007/151561, US-A-3489144, US-A-2007/017516 et US-A-2020/297964.

Dans ce contexte, un problème est de pourvoir éviter tout ou partie des problèmes susmentionnés en proposant une installation de fourniture de gaz respiratoire améliorée, typiquement d'un gaz contenant de l'oxygène, fonctionnant en circuit fermé, de manière à pouvoir éliminer efficacement le CO₂ présent dans les gaz expirés par le patient tout en minimisant les pertes d'oxygène en recyclant la majeure partie de l'oxygène présent dans les gaz expirés.

Une solution selon l'invention concerne une installation de fourniture de gaz respiratoire à un utilisateur, i.e. un patient (P), comprenant :
- au moins une source de gaz pour fournir un gaz respiratoire contenant de l'oxygène à un réservoir-tampon,
- une ligne principale d'acheminement de gaz reliant fluidiquement le réservoir-tampon à une interface respiratoire,
- une ligne de récupération de gaz reliant fluidiquement l'interface respiratoire à un système de purification de gaz et
- une ligne de recyclage de gaz reliant fluidiquement ledit système de purification de gaz au réservoir-tampon.

Selon l'invention, le système de purification de gaz comprend au moins un module de perméation, c'est-à-dire un ou plusieurs dispositifs de perméation.

L'installation de fourniture de gaz respiratoire de l'invention comprend en outre un système générateur de gaz frais comprenant un circuit de gaz interne relié fluidiquement audit au moins un module de perméation et à la source de gaz, le système générateur de gaz frais comprenant une vanne de contrôle, un dispositif à orifice calibré et un dispositif à venturi, agencés en série sur ledit circuit de gaz interne.

Selon le mode de réalisation considéré, l'installation de fourniture de gaz peut comprendre l'une ou plusieurs des caractéristiques suivantes:
- un dispositif régulateur de précision est agencé sur le circuit de gaz interne, entre la vanne de contrôle et le dispositif à orifice calibré.
- la vanne de contrôle est une vanne à actionnement manuel, i.e. une vanne manuelle.
- le dispositif à venturi est raccordé fluidiquement à une sortie du dispositif à orifice calibré.
- ladite sortie du dispositif à orifice calibré est en communication fluidique avec un volume interne situé en aval d'un col de venturi du dispositif à venturi.
- le diamètre de sortie, mesuré au niveau de la sortie, du dispositif à orifice calibré est inférieur à 500 µm, typiquement compris entre 50 µm et 400 µm.
- le dispositif à venturi comprend un port de sortie relié fluidiquement au module de perméation, via un conduit d'amenée de gaz frais.
- le (les) module de perméation comprend des fibres membranaires creuses agencées en parallèle les unes des autres.
- il comprend un seul module de perméation.
- selon un autre mode de réalisation, il comprend plusieurs modules de perméation agencés en série, en parallèle ou en cascade.
- la (ou les) source de gaz fournit un gaz respiratoire contenant plus de 80% d'oxygène, de préférence de l'oxygène pur.
- la source de gaz est reliée fluidiquement au réservoir-tampon via une ligne de fourniture de gaz, telle une canule ou un conduit de gaz.
- l'installation est du type à circuit fermé.
- une valve d'inhalation unidirectionnelle est agencée dans la ligne principale d'acheminement de gaz configurée pour autoriser la circulation de gaz respiratoire uniquement dans le sens allant du réservoir-tampon vers l'interface respiratoire.
- la source de gaz comprend une bouteille d'oxygène comprimé, c'est-à-dire à une pression d'au moins 100 bar, de préférence au moins 150 bar (bar absolus), i.e. pression mesurée lorsque la bouteille est pleine de gaz.
- l'interface respiratoire est configurée pour administrer le gaz respiratoire à l'utilisateur, lors de chaque phase inspiratoire dudit utilisateur.
- la ligne de récupération de gaz est configurée pour récupérer et acheminer au moins une partie du mélange gazeux CO₂/O₂ expiré par l'utilisateur se retrouvant dans l'interface respiratoire, lors de chaque phase expiratoire de l'utilisateur.
- la ligne principale d'acheminement de gaz comprend un (ou des) passage ou conduit de gaz ou analogue.
- le système de purification de gaz est alimenté en mélange gazeux CO₂/O₂ expiré par la ligne de récupération de gaz.
- le mélange gazeux CO₂/O₂ expiré contient au moins 90% d'oxygène et moins de 10% de CO₂, par exemple de l'ordre de 95% d'O₂ et de l'ordre de 5% de CO₂, et des impuretés éventuelles (N₂, Ar, H₂O...)
- le module de perméation est configuré pour éliminer au moins une partie du CO₂ contenu dans le mélange gazeux CO₂/O₂ expiré et obtenir un gaz purifié contenant essentiellement de l'O₂.
- la ligne de recyclage de gaz achemine un gaz purifié contenant essentiellement de l'oxygène provenant du module de perméation jusqu'au réservoir-tampon.
- l'interface respiratoire est ou comprend un masque respiratoire, en particulier un masque facial, i.e. bucco-nasal, couvrant le nez et la bouche de l'utilisateur.
- l'interface respiratoire est un masque respiratoire comprenant un orifice d'entrée d'oxygène pour fournir le gaz riche en oxygène provenant du réservoir-tampon et un orifice de sortie de gaz expiré pour évacuer le mélange gazeux expiré par l'utilisateur, c'est-à-dire les gaz expirés enrichis en CO₂.
- le masque respiratoire comprend un coussin souple venant en contact et assurant une étanchéité fluidique avec le visage de l'utilisateur.
- le masque respiratoire comprend un corps de masque, par exemple en polymère rigide, formant une coque délimitant une enceinte pour le gaz.
- le coussin souple est couplé mécaniquement au corps de masque, de préférence de manière détachable.
- le coussin souple comprend un passage central au sein duquel viennent se loger le nez et la bouche de l'utilisateur, lorsqu'il porte le masque facial.
- l'utilisateur respire le gaz contenu dans l'enceinte, c'est-à-dire inspire et expire le gaz dans l'enceinte du corps de masque.
- l'orifice d'entrée d'oxygène et l'orifice de sortie de gaz expiré sont aménagés dans le corps de masque.
- la ligne principale d'acheminement de gaz et/ou la ligne de récupération de gaz et/ou la ligne de recyclage de gaz sont ou comprennent une ou des conduites de gaz souples ou analogues, c'est-à-dire des tuyaux flexibles, par exemple en polymère.
- la ligne principale d'acheminement de gaz et/ou la ligne de récupération de gaz, respectivement, viennent se raccorder au corps de masque de manière à être en communication fluidique avec l'orifice d'entrée et/ou l'orifice de sortie de gaz expiré, respectivement.
- la ligne principale d'acheminement de gaz est raccordée fluidiquement à l'orifice d'entrée de l'interface respiratoire de manière à introduire du gaz respiratoire dans l'interface respiratoire.
- la ligne de récupération de gaz est raccordée fluidiquement à l'orifice de sortie de gaz expiré de l'interface respiratoire de manière à extraire du gaz respiratoire de l'interface respiratoire.
- le gaz purifié acheminé par la ligne de recyclage de gaz contient une proportion (i.e. teneur) d'oxygène inférieure ou égale au gaz provenant de la source de gaz, i.e. de l'oxygène pur.
- le réservoir-tampon comprend un ballon flexible, un soufflet ou analogue.
- le réservoir-tampon comprend une valve d'échappement permettant d'évacuer à l'atmosphère tout excès de gaz, lorsque le réservoir-tampon est plein de gaz.
- le réservoir-tampon est dimensionné pour contenir de 0.5 à 10 litres de gaz (à l'état non-comprimé).
- le réservoir-tampon est configuré pour alimenter la ligne principale d'acheminement de gaz avec le gaz respiratoire, i.e. un gaz contenant essentiellement de l'oxygène.
- une valve d'expiration unidirectionnelle, agencée dans la ligne de récupération de gaz, est configurée pour n'autoriser la circulation du gaz que dans le sens allant de l'interface respiratoire vers le (les) module de perméation, i.e. elle empêche toute remontée de gaz dans le sens inverse.
- la bouteille d'oxygène est munie d'un robinet à détendeur intégré (RDI).
- la bouteille d'oxygène est équipée d'un RDI comprenant deux ports de sortie comprenant un port de sortie en débit et un port de sortie en pression.
- le port de sortie en débit est configuré pour délivrer différents débits gazeux compris entre 0 et 15 L/min (i.e. débit d'oxygène).
- le port de sortie en pression est configuré pour fournir une pression gazeuse fixe, de préférence comprise entre 1,5 et 8 bar abs, typiquement de l'ordre de 4 bar abs (i.e. pression d'oxygène).
- le RDI comprend un dispositif sélecteur de débit configuré pour permettre à l'utilisateur de sélectionner un débit désiré (i.e. débit d'oxygène).
- le réservoir-tampon comprend un premier port d'entrée en communication fluidique avec le port de sortie en débit du RDI, via une ligne de fourniture de gaz, telle une canule, un conduit de gaz ou analogue.
- le réservoir-tampon comprend un second port d'entrée en communication fluidique avec le (les) module de perméation, via la ligne de recyclage de gaz.
- le réservoir-tampon comprend un port de sortie en communication fluidique avec l'interface respiratoire, via la ligne principale d'acheminement de gaz.
- le (les) module de perméation comprend :
   . un port d'alimentation pour recevoir le gaz à purifier (i.e. contenant O₂ et CO₂), c'est-à-dire le gaz expiré ;
   . un port de rétentat fournissant le gaz purifié, c'est-à-dire essentiellement de l'oxygène (i.e. O₂ n'ayant pas perméé au travers des fibres membranaires) ;
   . un port de perméat fournissant le gaz à éliminer, c'est-à-dire essentiellement du CO₂ (i.e. ayant perméé) ; et
   . un port de gaz frais alimenté en gaz frais (i.e. essentiellement de l'O₂), c'est-à-dire du gaz frais provenant du système générateur de gaz frais.
- le port d'alimentation est relié fluidiquement à la ligne de récupération de gaz.
- le port de rétentat est relié fluidiquement à la ligne de recyclage de gaz.
- le port de perméat est relié fluidiquement à l'atmosphère ambiante.
- le port de gaz frais est relié fluidiquement au conduit d'amenée de gaz frais.
- le (les) module de perméation comprend au moins plusieurs dizaines ou centaines de fibres creuses agencées en parallèle les unes des autres.
- les fibres creuses agencées sont agencées dans une enveloppe périphérique, i.e. un boitier ou analogue.
- chaque fibre creuse a une structure générale tubulaire formant une membrane de séparation par perméation, c'est-à-dire une membrane perméable tubulaire.
- chaque fibre creuse membranaire présente une sélectivité membranaire supérieure pour les molécules de CO₂ que pour celles d'O₂, c'est-à-dire que leur membrane est traversée préférentiellement par les molécules de CO₂ (i.e. perméât) mais, qu'à l'inverse, elle retient celles d'O₂ (i.e. retentât) qui restent dans le lumen des fibres creuses membranaires.
- chaque fibre creuse membranaire comprend une paroi tubulaire formée d'une couche mince de quelques µm d'épaisseur, d'un matériau perméable.
- le matériau perméable est par exemple de type polydiméthylsiloxane (PDMS) ou polyimide (PI).
- le (les ou chaque) module de perméation comprend en outre une première chambre en communication fluidique avec le port d'alimentation et une deuxième chambre en communication fluidique avec le port retentât.
- la première chambre et la deuxième chambre sont fluidiquement isolées l'une de l'autre.
- la première chambre (i.e. chambre amont) et la deuxième chambre (i.e. chambre aval) sont séparées l'une de l'autre par une troisième chambre (i.e. chambre intermédiaire), c'est-à-dire que la troisième chambre est prise en sandwich entre les première et deuxième chambres.
- la troisième chambre est en communication fluidique avec le port de perméat et le port de gaz frais.
- chaque fibre creuse comprend une extrémité proximale par laquelle entre le gaz à purifier (i.e. gaz expirés avec O₂ et CO₂) et une extrémité distale par laquelle ressort le gaz de retentât (i.e. principalement de l'O₂).
- les extrémités proximales des fibres creuses débouchent dans la première chambre.
- les extrémités distales des fibres creuses débouchent dans la deuxième chambre.
- les fibres creuses traverses des parois étanches, agencées au niveau des extrémités proximales et distales, assurant une étanchéité fluidique entre les première, deuxième et troisième chambres du module de perméation.
- la première chambre, la deuxième chambre et la troisième chambre sont agencées dans l'enveloppe périphérique.
- les fibres creuses s'étendent entre la première chambre et la deuxième chambre en traversant la troisième chambre.
- la vanne de contrôle est configurée pour être mobile, typiquement manœuvrable manuellement par l'utilisateur, entre au moins une position d'ouverture autorisant une circulation du gaz dans le circuit de gaz interne et une position de fermeture empêchant toute circulation de gaz dans le circuit de gaz interne.
- la vanne de contrôle comprend un organe de manœuvre, tel un bouton rotatif, servant à manœuvrer manuellement la vanne entre au moins les positions d'ouverture et de fermeture.
- le dispositif à orifice calibré comprend un diamètre de sortie inférieur à 500 µm, de préférence d'au moins 50 µm, par exemple de l'ordre de 100 µm.
- le dispositif à venturi comprend un corps principal définissant un volume interne et un col de venturi.
- le dispositif à venturi comprend un orifice d'admission de gaz en communication fluidique avec l'atmosphère.
- l'orifice d'admission de gaz est agencé au niveau du col de venturi.
- l'orifice d'admission de gaz présente une aire de section comprise entre 0.5 et 10 mm², par exemple de l'ordre de 5 mm² environ.
- le corps principal du dispositif à venturi comprend un port de sortie relié fluidiquement au conduit d'amenée de gaz frais.
- le dispositif régulateur de précision est configuré pour délivrer une pression gazeuse fixe donnée, de préférence comprise entre 1 et 2 bar relatif, par exemple de l'ordre de 1.5 bar relatif.
- la vanne de contrôle, le dispositif à orifice calibré, le dispositif à venturi et, s'il est présent, le dispositif régulateur de précision, sont agencés dans un boitier commun.

Il est à noter que, dans le cadre de la présente invention, on utilise indifféremment la terminologie « le gaz expiré » ou « les gaz expirés » (i.e. singulier ou pluriel) pour désigner le mélange gazeux rejeté par les poumons du patient, lequel contient de l'O₂ et du CO₂, et généralement de la vapeur d'eau éventuellement d'autres constituants, comme de l'argon ou de l'azote.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de fourniture de gaz respiratoire à circuit fermé selon la présente invention.
Fig. 2 schématise un mode de réalisation d'un module de perméation de l'installation de Fig. 1.
Fig. 3 schématise un mode de réalisation du système générateur de gaz frais de l'installation de Fig. 1.

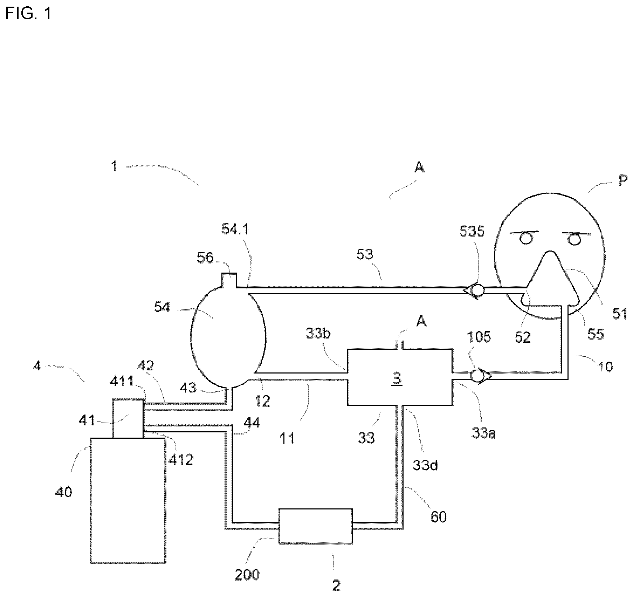

Fig. 1 schématise un mode de réalisation d'une installation de fourniture de gaz respiratoire 1, à circuit fermé, selon la présente invention incluant un système de recyclage des gaz expirés.

L'installation 1 comprend une source de gaz 4 pour fournir un gaz respiratoire contenant de l'oxygène ; une ligne principale d'acheminement de gaz 53, aussi appelée circuit patient ; une interface respiratoire 51, tel un masque facial, pour administrer le gaz respiratoire à l'utilisateur ou patient P, lors de ses phases inspiratoires ; un système de purification 3 des gaz expirés ; et un système de génération de gaz frais 2.

La source de gaz 4 peut être par exemple une bouteille d'oxygène 40 comprimé munie d'un robinet détendeur intégré 41 ou RDI muni de deux ports, à savoir un port de sortie en débit 411 permettant de délivrer différents débits que l'on peut choisir au moyen d'un sélecteur de débit, par exemple des débits de 1 à 15 L/min, et un port de sortie en pression 412 permettant une connexion dite « haute pression », pour fournir une pression gazeuse fixe, typiquement de l'ordre de 5 bar abs.

De préférence, le sélecteur de débit permet de régler le débit d'O₂ délivré par le RDI de manière à fournir un débit faible, à savoir de l'ordre de 1, 2 ou 3 L/min par exemple, dans le cadre de la présente invention. Le sélecteur de débit peut comprendre par exemple un volant rotatif coopérant avec un disque à orifices calibrés.

L'installation 1 comprend en outre un réservoir-tampon 54 servant de réserve de gaz respiratoire (i.e. d'oxygène). De préférence, il a une capacité de l'ordre de 0.5 à 10 L (équivalent en eau), préférentiellement de l'ordre de 3 L.

La ligne principale d'acheminement de gaz 53 ou circuit patient, tel un tuyau ou conduit flexible, relie fluidiquement un port de sortie 54.1 du réservoir-tampon 54 à un orifice ou port d'entrée 52 de l'interface respiratoire 51 de manière à alimenter le patient P en gaz respiratoire, typiquement de l'oxygène (O₂) provenant du réservoir-tampon 54.

Lorsque le patient P respire, le réservoir-tampon 54 qui est rempli de gaz, i.e. d'oxygène, satisfait la demande instantanée en gaz du patient P en lui fournissant du gaz.

Le remplissage du réservoir-tampon 54 avec de l'oxygène se fait via un premier port d'entrée 43, auquel est relié fluidiquement le port de sortie en débit 411 du RDI 41, par l'intermédiaire d'une ligne de fourniture de gaz 42, telle une canule ou un conduit, par exemple en polymère, silicone ou analogue.

Par ailleurs, une valve d'inhalation unidirectionnelle 535 est agencée dans la ligne principale d'acheminement de gaz 53, c'est-à-dire entre le réservoir-tampon 54 et l'interface respiratoire 51. Cette valve unidirectionnelle d'inhalation 535 permet une circulation du gaz au sein de la ligne principale d'acheminement de gaz 53 uniquement dans le sens allant du réservoir tampon 54 vers l'interface respiratoire 51, c'est-à-dire que le gaz ne peut la traverser que dans ce sens et qu'elle interdit toute remontée ou cheminement du gaz en sens inverse.

Pendant le fonctionnement de l'installation 1, le gaz expiré par le patient P, qui est riche en CO₂ mais contient encore de l'oxygène non-métabolisé, ressort de l'interface respiratoire 51 par un port ou orifice de sortie 25 aménagé dans l'interface respiratoire 51, avant d'être récupéré et convoyé par une ligne de récupération de gaz 10 formant un circuit expiratoire, tel un conduit ou tuyau de gaz flexible, connecté fluidiquement au port de sortie 25 de l'interface respiratoire 51.

Cette ligne de récupération de gaz 10 permet de récupérer et acheminer au moins une partie du mélange gazeux expiré se retrouvant dans l'interface respiratoire 51, lors des phases expiratoires de l'utilisateur P. Ce gaz expiré contient différents composés gazeux, majoritairement de l'O₂ et du dioxyde de carbone (CO₂), mais aussi des composés secondaires inévitables, comme de la vapeur d'eau (H₂O), de l'azote (N₂), voire de l'argon (Ar).

Dans tous les cas, le mélange gazeux expiré contient une proportion de CO₂ non nulle puisque résultant de la respiration du patient et des échanges pulmonaires entre O₂ et CO₂, typiquement une proportion de CO₂ de l'ordre de 5 % vol.

Le flux de gaz expiré est acheminé par la ligne de récupération de gaz 10 jusqu'à un système de purification du gaz expiré, à savoir ici un système de purification membranaire des gaz expirés comprenant un (ou plusieurs) module de perméation 33.

Le/chaque (ou les) module de séparation 33 du système de purification 3 des gaz expirés sert à purifier le flux de gaz expiré par la patient (contenant O₂ et CO₂) pour en éliminer le CO₂ par perméation membranaire et à l'évacuer vers l'atmosphère ambiante A, comme décrit ci-après. Le gaz purifié produit par le module de perméation 33 contient essentiellement de l'O₂ (i.e. > 98%) et une proportion faible ou négligeable de CO₂ et/ou des impuretés inévitables.

Autrement dit, le (ou les) module de perméation 33 du système de purification 3 des gaz expirés permet d'éliminer la majeure partie, préférentiellement la (quasi-)totalité du CO₂ qui est contenue dans le mélange gazeux expiré par le patient P et d'obtenir ainsi un gaz purifié contenant essentiellement de l'oxygène, voire des impuretés inévitables, comme de la vapeur d'eau, de l'azote et/ou de l'argon en proportions négligeables.

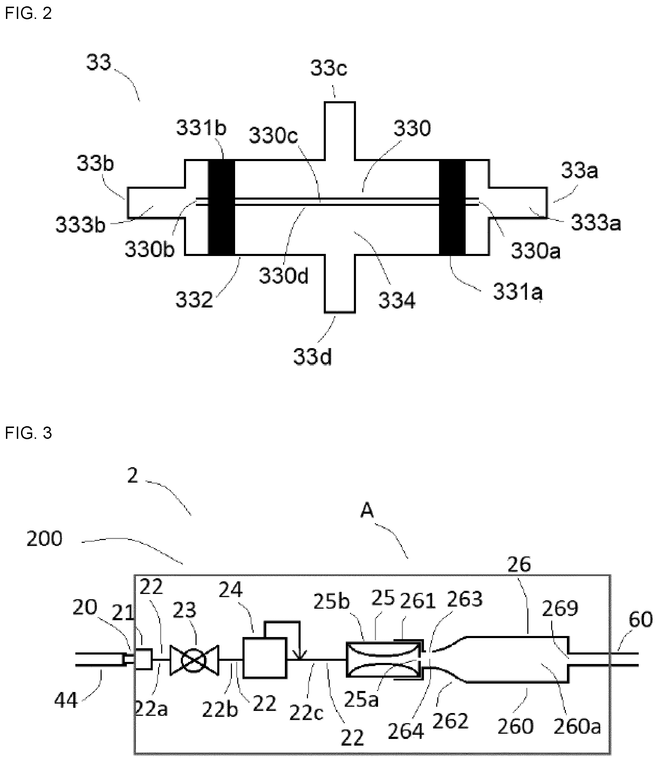

Dans le mode de réalisation de Fig. 1 et Fig. 2, le système de purification membranaire 3 de l'installation 1 comprend un seul module de perméation 33 ; toutefois, dans d'autres modes de réalisation, plusieurs modules de perméation 33 peuvent être mise en œuvre et, selon le cas, ils peuvent être agencés en parallèles, en série ou en cascade.

Par ailleurs, une valve d'expiration unidirectionnelle 105, agencée dans la ligne de récupération de gaz 10, n'autorise la circulation du gaz que dans le sens allant de l'orifice de sortie 25 de l'interface respiratoire 51 au module de perméation 33, en empêchant toute remontée de gaz dans le sens inverse, c'est-à-dire vers l'interface 51.

L'installation 1 de l'invention comprend aussi, agencée en aval du système de purification 3 des gaz expirés, c'est-à-dire en aval du (des) module de perméation 33, une ligne de recyclage de gaz 11 reliée fluidiquement, d'une part, au module de perméation 33 et, d'autre part, au réservoir-tampon 54, de manière à recueillir le gaz purifié sortant du (des) module de perméation 33 et à l'acheminer jusqu'au réservoir 54. Le gaz purifié, i.e. essentiellement de l'oxygène, pénètre dans le réservoir-tampon 54, via un second port d'entrée 12 auquel est raccordée fluidiquement la ligne de recyclage de gaz 11.

En outre, le réservoir-tampon 54 est équipé d'une valve d'échappement 56 configurée pour évacuer à l'atmosphère ambiante A, tout excès de gaz, i.e. O₂, au sein du réservoir-tampon 24, lorsqu'il est plein, c'est-à-dire d'éviter toute surpression gazeuse dans le réservoir-tampon 54.

Utiliser un système de purification 3 des gaz expirés comprenant un (ou des) module de perméation 33 est avantageux car il permet de purifier efficacement et recycler les gaz expirés en les débarrassant du CO₂ et préférentiellement de la vapeur d'eau (H₂O) qu'ils contiennent, au lieu de les rejeter à l'atmosphère et ainsi d'éviter de gaspiller l'oxygène expiré par le patient qui s'y trouve encore. Ceci permet dès lors de limiter le débit d'oxygène fourni par la source d'O₂ 4 puisque l'oxygène recyclé est (ré)introduit dans le réservoir-tampon 54.

Ainsi, par exemple, si l'on considère que la consommation métabolique d'O₂ d'un être humain est de l'ordre de 0.5 L/min, qui correspond à une ventilation minute de 10 L/min, i.e. 10L de gaz (100% d'O₂) inhalé par le patient pendant une minute, une quantité d'environ 9.5 L d'O₂ est expiré, le reste, i.e. environ 0.5 L/min étant formé de CO₂ et de vapeur d'eau.

L'installation 1 de l'invention étant à circuit fermé, permet de récupérer une quantité non-négligeable d'O₂. En réglant le RDI 41 de la bouteille 40 à un débit de 1, 2 ou 3 L/min et en recyclant l'O₂ en le mélangeant avec l'O₂ provenant de la bouteille 40, le débit d'O₂ récupéré excède la ventilation minute du patient.

Fig.2 schématise un mode de réalisation d'un module de perméation 33 du système de purification 3 des gaz expirés servant à éliminer le CO₂ de l'installation à circuit fermé de Fig. 1. Le (chaque) module de perméation 33 comprend une enveloppe 332 externe formant une carcasse en matériau rigide, par exemple en acier ou en polymère, par exemple de type polycarbonate.

Le (chaque) module de perméation 33, typiquement l'enveloppe externe 332, présente 4 ports ou orifices servant aux échanges gazeux, à savoir un port d'alimentation 33a par lequel entrent les gaz expirés (i.e. O₂/CO₂) provenant de la ligne de récupération de gaz 10 ; un port de rétentat 33b d'où une partie des gaz expirés (i.e. O₂) ressort du module de perméation 33 (i.e. en étant retenu, c'est-à-dire sans avoir perméée) ; un port de perméat 33c d'où une partie du gaz ayant perméé (i.e. CO₂) ressort du module de perméation 33 ; et un port de gaz frais 33d alimenté en gaz frais (i.e. O₂).

Le port d'alimentation 33a débouche dans une première chambre 333a du module de perméation 33, aussi appelée chambre amont ou chambre d'alimentation. Le module de perméation 33 comprend par ailleurs une deuxième chambre 333b, aussi appelée chambre aval ou chambre de retentât, communiquant fluidiquement avec le port de rétentat 33b,

Une troisième chambre 334, aussi appelée chambre intermédiaire ou chambre de perméat, comprenant le port de perméat 33c et le port de gaz frais 33d, est agencée entre la première chambre 333a et la deuxième chambre 333b du module de perméation 33.

A l'intérieur de l'enveloppe externe 332 du module de perméation 33 se trouvent des membranes de perméation de type fibres creuses 330 agencées en parallèle les unes des autres. Chaque fibre creuse 330 présente un canal interne ou lumen 330c dans lequel les gaz expirés peuvent circuler.

Chaque fibre creuse 330 est délimitée par une membrane périphérique 330d perméable constituant la paroi périphérique de chaque fibre creuse 330.

Autrement dit, chaque fibre creuse 330 a une structure générale tubulaire formant une membrane de séparation par perméation, c'est-à-dire une membrane perméable tubulaire, présentant des sélectivités différentes pour différents composés gazeux, c'est-à-dire qu'elle laisse passer certaines molécules (typiquement ici le CO₂) ou, à l'inverse, retient d'autres molécules (typiquement ici l'O₂), comme expliqué ci-après.

Plus précisément, la membrane périphérique 330d de chaque fibre creuse 330 comprend une paroi tubulaire formée d'une couche mince, typiquement de quelques µm d'épaisseur, de matériau perméable comme par exemple, selon les caractéristiques de perméabilité recherchées, du polydiméthylsiloxane (PDMS), i.e. élastomère de silicone, ou du polyimide (PI).

Chaque fibre creuse 330 est mécaniquement maintenue par des parois étanches ou scellements 331a, 331b agencés dans ses régions d'extrémités, par exemple des bouchons ou analogues, assurant une étanchéité fluidique entre les première, deuxième et troisième chambres 333a, 333b et 334 du module de perméation 33.

Les parois étanches ou scellements peuvent être par exemple de type résine époxy.

La composition des fibres creuses 330, à savoir le matériau formant la membrane périphérique 330d, est choisie pour permettre de séparer sélectivement par perméation une (ou plusieurs) espèce(s) ou composé(s) gazeux présent(s) dans le mélange gazeux d'alimentation circulant dans le lumen de ladite fibre creuse 330.

Les gaz expirés, provenant de la ligne de récupération de gaz 10 et alimentant la chambre 333a du (de chaque) module de perméation 33, passent dans le lumen 330c de chaque fibre creuse 330 en y entrant par une extrémité proximale 330a (i.e. entrée proximale) en communication fluidique avec la chambre amont 333a du module de perméation 33.

Ainsi, une (ou des) molécule gazeuse donnée, telle celle de CO₂, peut traverser par perméation la membrane 330d de chaque fibre 330 et donc passer du canal interne ou lumen 330c de chaque fibre creuse 330 à la troisième chambre 334 du module de perméation 33. Le nombre de molécules pénétrant dans chaque fibre creuse 330 et traversant la membrane 330d dépend des propriétés de cette membrane externe 330d et du gradient de pression existant entre le canal interne 330c de la fibre creuse 330 et la troisième chambre 334 du module de perméation 33, pour une molécule donnée.

Par exemple, pour un mélange 95% O₂/5% CO₂ à pression atmosphérique présent dans le canal interne 330c de la fibre creuse, la pression partielle en CO₂ est d'environ 50 mbar, alors que celle en O₂ sera de 950 mbar (le cumul des deux pressions partielles étant égal a la pression atmosphérique, i.e. 1000 mbar).

Si le media présent dans la troisième chambre 334 du module de perméation 33 est de l'air, alors il contient alors environ 21% d'O₂ et une quantité négligeable de CO₂, donc une pression partielle en O₂ est de 210 mbar et une pression partielle en CO₂ négligeable, i.e. environ 0 mbar.

Dès lors, le gradient de pression, dans le sens canal interne 330c vers troisième chambre 334, existant au niveau de la membrane 330d est de 740 mbar pour l'O₂ et 50mb pour le CO₂. Bien entendu, plus la pression au sein du canal interne 330c de la fibre creuse 330 est importante (par exemple sous l'effet d'une compression), plus les pressions partielles des gaz contenus dans le canal interne 330c sont importantes.

Dès lors, afin de favoriser la perméation des molécules de CO₂ par rapport aux autres, i.e. à celles d'oxygène, on utilise une membrane 330d formée d'un matériau ayant une sélectivité préférentiellement plus élevée pour le CO₂, par exemple en PI-C. En effet, la sélectivité CO₂/O₂ d'une membrane de type PI-C est de 10 :1, c'est-à-dire que le taux de perméation des molécules de CO₂ est 10 fois plus élevé que celui des molécules de O₂. Le PI-C est donc un matériau qui convient à une séparation par perméation des molécules de CO₂ contenues dans les gaz expirés contenant les composés O₂ et CO₂. Bien entendu, d'autres matériaux que PI-C peuvent être utilisés pour former la membrane 330d de chaque fibre creuse 330 dès lors que leur sélectivité est adéquate, c'est-à-dire convient à la séparation désirée.

De là, en supposant que 1 unité de gaz expirés (i.e. mélange gazeux 95%O₂/5% CO₂) entre dans le module de séparation de CO₂ 33 et qu'un gradient de pression existe entre le lumen 330c de chaque fibre creuse 330 et la troisième chambre 334, une partie β des gaz expirés va traverser la membrane externe 330d, c'est-à-dire le perméat du module membranaire, alors que le reste, i.e. le rétentat, qui est égal à (1-β), va sortir de chaque fibre creuse 330 par son extrémité distale 330b (i.e. sortie distale).

Du fait de la sélectivité plus élevée de la membrane externe 330d pour les espèces CO₂, le débit de perméat β est enrichi en CO₂ qui traverse plus facilement la membrane externe 330d. Ce débit de perméat β contient également de l'O₂ en fonction du gradient de pression en O₂ existant entre le lumen 330c de chaque fibre creuse 330 et la troisième chambre 334, mais en quantité faible ou limitée du fait de la sélectivité de la fibre creuse 330 qui est supérieure pour le CO₂ que pour l'O₂. Le flux de rétentat (1-β) est quant à lui enrichi en O₂, donc dépourvu de CO₂.

Préférentiellement, le module de perméation 33 comprend plusieurs centaines ou plusieurs milliers de fibres 330, agencées en parallèles les unes des autres. Utiliser un grand nombre de fibres 330 permet d'augmenter la capacité de perméation tout en minimisant les pertes de charges.

Dans le cadre de la présente invention, le (les) module de perméation 33 du système de purification 3 est dimensionné et/ou configuré (i.e. un nombre de fibres creuses 330) pour qu'un patient P puisse expirer au travers du module de perméation 33 sans éprouver de gêne particulière due aux pertes de charges générées, par exemple conçu pour engendrer une perte de charge jusqu'à environ 2 mbar pour un débit expiré instantané de 60L/min.

En fonction des propriétés de la membrane 330d formant les fibres 330, la troisième chambre 334 du module de perméation 33 peut recueillir une forte concentration en CO₂, qui dépend néanmoins du gradient de pression partielle du CO₂ entre le conduit interne 330c de la fibre 330 et la troisième chambre 334.

Ainsi, sans action spécifique, la pression partielle en CO₂ (et donc sa concentration) dans la troisième chambre 334 va progressivement atteindre la pression partielle en CO₂ régnant dans le conduit interne 330c, c'est-à-dire 50 mbar environ, et ce, jusqu'à l'équilibre. Le taux de perméation du CO₂ au travers de la membrane 330d (du conduit interne 330c à la troisième chambre 334) va dès lors progressivement diminuer et devenir nul lorsque cet équilibre est atteint.

Le CO₂ contenu dans les gaz expirés alimentant le module de séparation de CO₂ 33 ne pourra alors plus être évacué. De ce fait, le module de séparation de CO₂ 3 dispose d'un port de gaz frais 33d relié fluidiquement à la troisième chambre 334 et au port de perméat 33c de sorte que tout gaz entrant par le port de gaz frais 33d se répand dans la troisième chambre 334 pour ensuite sortir par le port de perméat 33c, débouchant à l'atmosphère ambiante A. Le port de gaz frais 33d est raccordé à un conduit d'amenée de gaz frais 60, lui-même relié à un système générateur de gaz frais 2, comme illustré en Fig.1.

Comme illustré sur Fig. 1 et Fig. 3, le système générateur de gaz frais 2, aussi appelé générateur de gaz frais, est raccordé, via un port d'entrée 21, à un conduit flexible 44 raccordé fluidiquement à la source de gaz 4, en particulier au port de sortie en pression 412 de la source de gaz 4 fournissant une pression gazeuse fixe, typiquement de l'ordre de 5 bar abs, c'est-à-dire du gaz « sous pression ».

Le raccordement au port d'entrée 21 se fait via des moyens de raccordement amont 20, tel un connecteur ou analogue, de préférence à connexion rapide qui permet à un utilisateur de pouvoir connecter et déconnecter le flexible de raccordement 44 de manière simple et rapide.

Le port d'entrée 21 met en relation le flexible de raccordement 44 alimenté par le gaz sous pression provenant de la source de gaz 4, i.e. une bouteille 40, au circuit de gaz interne 22, en particulier à la ligne d'admission de gaz dudit circuit de gaz interne 22, qui est agencé dans le boitier rigide 200 d'un système générateur de gaz frais 2.

Le circuit de gaz interne 22 comprend plusieurs dispositifs agencés en série, c'est-à-dire successivement, en particulier une vanne de contrôle 23, typiquement une vanne manuelle, c'est-à-dire à actionnement manuel, un dispositif régulateur de précision 24 optionnel, un dispositif à orifice calibré 25 et un dispositif à venturi 26. Le gaz sous pression, à savoir de l'O₂ à environ 4 bar abs, circule dans le circuit de gaz interne 22 dans le sens allant du port d'entrée 21 vers le conduit d'amenée de gaz frais 60 et passe au travers de ces différents dispositifs.

La vanne de contrôle 23 est immédiatement en aval du port d'entrée 21 du circuit de gaz interne 22.

Dans le mode de réalisation de Fig. 3 décrit la vanne de contrôle 23 est manuelle. Elle est manœuvrable par l'utilisateur entre une position d'ouverture et une position de fermeture, et inversement, via un organe de manœuvre, tel un bouton rotatif (non montré), de manière à contrôler la circulation de gaz dans le circuit de gaz interne 22. Toutefois, selon un autre mode de réalisation (non représenté), la vanne de contrôle 23 n'est pas à actionnement manuelle mais à commande électrique via un système de commande dédié.

Optionnellement, un dispositif régulateur de précision 24 peut être agencé sur le circuit de gaz interne 22, en aval de la vanne de contrôle 23. Ce dispositif régulateur de précision 24 est configuré pour délivrer une pression fixe donnée, comme décrit ci-après, par exemple comprise entre 1 et 2 bar relatif, par exemple de l'ordre de 1.5 bar relatif. On peut utiliser par exemple le régulateur de précision commercialisé par la société Beswick^{®} sous la référence commerciale PRD.

Lorsque la vanne de contrôle 23 est en position de fermeture, le premier tronçon 22a (i.e. tronçon d'entrée) du circuit de gaz interne 22, qui fait office de ligne d'admission et qui est situé en amont de la vanne manuelle 23, est soumis au gaz sous pression (i.e. env. 4 bar abs) entrant dans le circuit 22 par son port d'entrée 21, alors qu'un second tronçon 22b (i.e. tronçon intermédiaire) situé entre la vanne manuelle 23 et le régulateur de précision 24, et un troisième tronçon 22c (i.e. tronçon aval) situé en aval du régulateur de précision 24, sont soumis à pression atmosphérique (i.e. 1 bar abs), comme visible sur Fig. 3.

Le dispositif à orifice calibré 25 est agencé sur le circuit de gaz interne 22, en aval de la vanne de contrôle 23 et du dispositif régulateur de précision 24. Le troisième tronçon 22c relie donc fluidiquement le dispositif régulateur de précision 24 au dispositif à orifice calibré 25, comme illustré en Fig. 3. Le diamètre de sortie du dispositif à orifice calibré 25, qui est situé au niveau de sa sortie 25a, est préférentiellement inférieur à 500 µm. On peut utiliser par exemple celui commercialisé par la société O'Keefe Control^{®}, sous la référence commerciale B4-SS, ayant un diamètre de sortie de l'ordre de 100 µm.

Le dispositif à orifice calibré 25 est couplé mécaniquement et fluidiquement à un dispositif à venturi 26, par exemple via un épaulement 261 du dispositif à venturi 26 venant se solidariser à la surface externe 25b du dispositif à orifice calibré 25, par exemple par vissage, montage en force ou tout autre technique.

Par ailleurs, le dispositif à venturi 26 comprend un corps principal 260 définissant un volume interne 260a, et un col de venturi 262 reliant l'épaulement 261 au corps principal 260. Il comprend en outre un orifice d'admission de gaz 263 en communication fluidique avec l'atmosphère A, par exemple porté par le col de venturi 262. L'orifice d'admission 263 peut revêtir différentes formes, telle que rectangulaire, circulaire, ou autre. De préférence, il présente une aire de section comprise entre 0.5 et 10 mm², par exemple de l'ordre de 5 mm² environ.

Le volume interne du col de venturi 262 du dispositif à venturi 26 comprend ou forme une chambre de venturi 264, sensiblement comprise entre la sortie 25a du dispositif à orifice calibré 25 et l'orifice d'admission 263. La sortie 25a du dispositif à orifice calibré 25 est en communication fluidique avec le volume interne 260a du corps principal 260 du dispositif à venturi 26, via le col de venturi 262.

Le corps principal 260 du dispositif à venturi 26 présente par ailleurs un orifice ou port de sortie 269 relié fluidiquement au conduit d'amenée de gaz frais 60.

L'orifice d'admission de gaz 263 et le port de sortie 269 sont en communication fluidique avec le volume interne 260a du corps principal 260 du dispositif à venturi 26.

Lorsque l'utilisateur manœuvre la vanne manuelle 23 pour la passer en position d'ouverture, la pression gazeuse se propage en aval de la vanne manuelle 23, via le second tronçon 22b, jusqu'au régulateur de précision 24, lorsqu'il est présent. Le régulateur de précision 24 génère alors une pression de détente utile inférieure à la pression du port de sortie en pression 412 de la source de gaz 4, de préférence une pression de détente utile comprise entre 1 et 2 bar, par exemple de l'ordre de 1.5 bar. La pression de détente utile se propage alors dans le troisième tronçon 22c du circuit de gaz interne 22, qui est situé en amont de l'orifice calibré 25.

Or, il existe une relation entre la pression en amont du dispositif à orifice calibré 25, i.e. dans le troisième tronçon 22c, et le débit sortant de celui-ci, via sa sortie 25a. Du fait de la faible dimension (i.e. < 500 µm) du diamètre de sortie de la sortie 25a, par exemple de l'ordre de 100 µm, l'orifice calibré 25 génère un fluide à un débit volumique faible, par exemple de l'ordre de 0.2 L/min, et à haute vélocité, par exemple de l'ordre de 5 m/s, au niveau de sa sortie 25a. Ceci va créer une dépression dans la chambre venturi 264, laquelle va elle-même créer un différentiel de pression négatif entre la pression absolue régnant dans la chambre venturi 264, et la pression absolue, i.e. c'est-à-dire la pression atmosphérique, de l'air ambiant A en communication fluidique avec la chambre venturi 264 via l'orifice d'admission 263. Le différentiel de pression négatif créé engendre une aspiration d'un débit d'air provenant de l'atmosphère ambiante A via l'orifice d'admission 263. L'air aspiré contient notamment environ 20.9 vol.% d'O₂ (i.e. environ 21% d'O₂) et bien entendu de l'azote et de l'argon.

Le débit d'air entrant par l'orifice d'admission venturi 263 se mélange au débit d'O₂ délivré par l'orifice calibré 25. En dimensionnant le diamètre de sortie de l'orifice calibré 25 (au niveau de la sortie 25a) et l'orifice d'admission venturi 263, on peut obtenir un rapport important entre le débit sortant de l'orifice calibré 25 et le débit entrant par l'orifice d'admission venturi 263. Ce rapport peut être compris 50 et 200, par exemple de l'ordre de 100.

Le mélange gazeux ainsi crée (environ 21% d'O₂) a alors un débit de l'ordre de 20 L/min par exemple, et se propage alors dans le volume interne 260a du corps principal 260 du dispositif à venturi 26, puis dans le conduit d'amenée de gaz frais 60.

Le débit de gaz frais circulant dans le conduit d'amenée de gaz frais 60 va pénétrer dans le (les) module de séparation de CO₂ 33, via le port de gaz frais 33d, pour ensuite entrer dans la troisième chambre 334 du module de séparation de CO₂ 33 où règne initialement une concentration en CO₂ donnée, issue de la perméation du CO₂ au travers de la membrane 330d de la fibre 330.

Le débit de gaz frais va alors chasser le CO₂ présent dans la troisième chambre 334 vers l'atmosphère ambiante A au travers du port de perméat 33c. La pression partielle en CO₂ dans la troisième chambre 334 devient alors négligeable au regard de la pression partielle en CO₂ régnant dans le conduit interne 330c de la fibre 330, permettant alors au CO₂ contenu dans les gaz expirés de perméer à nouveau au travers de la membrane 330d des fibres 330 pour se retrouver dans la troisième chambre 334 et ensuite d'être éliminé vers l'atmosphère A, via le port de perméat 33c.

La quantité d'O₂ nécessaire pour alimenter le système générateur de gaz frais 2 est très faible, par exemple de l'ordre de 0.2 L/min, ce qui permet d'accroître l'autonomie de la bouteille d'oxygène, i.e. de la source de gaz 4.

Selon un autre mode de réalisation, on peut supprimer le régulateur de pression de précision 24 pour travailler directement avec la pression de sortie provenant du port de sortie en pression 412 (e.g. de l'ordre de 4 bar abs) et en adaptant alors le diamètre de l'orifice calibré 25, de manière à utiliser l'énergie pneumatique de la source de gaz 4 pour obtenir la fonction venturi. Dans ce cas, les deuxième et troisième tronçons 22b, 22c se confondent en un tronçon aval unique.

Dans tous les cas, on note qu'une partie de l'O₂ circulant dans le lumen 330c des fibres creuses 330 va traverser leur membrane externe 330d et se retrouver aussi dans la troisième chambre 334. Toutefois, du fait de leur sélectivité, la quantité de molécules d'O₂ traversant les membranes externes 330d reste modérée par, exemple de l'ordre de 1L/min, ce qui n'impacte pas significativement l'économie d'O₂ obtenue selon la présente invention.

Par ailleurs, en supposant que les gaz expirés contiennent un mélange 95% O₂/5% CO₂ et que le gaz frais entrant dans la chambre 334 du (des) module de séparation de CO₂ 33 du système de purification 3 est proche de l'air ambient, les fibres creuses 330 sont exposées à un gradient de pression entre la chambre 334 contenant une pression partielle en N₂ de l'ordre de 780 mb (78% vol.), et le lumen 330c des ces fibres creuses 330, ne contenant pas de N₂. Un tel gradient force une partie du N₂ à traverser la membrane 330d des fibres creuses 330, de la chambre 334 vers le lumen 330c des fibres creuses 330.

Toutefois, le N₂ est un gaz « lent » à diffuser dans les membranes et qui plus est le matériau utilisé à titre d'exemple dans le cadre de ladite invention présente une sélectivité encore plus faible vis-à-vis du N₂. Si la sélectivité CO₂/O₂ est de l'ordre de 10 comme décrit ci-avant, elle de l'ordre de 60 pour le N₂. Ainsi, la quantité de N₂ se retrouvant dans la chambre de rétentat 333b et circulant par la suite dans la ligne de recyclage de gaz 11, et entrant dans le réservoir-tampon 54 via son port d'entrée 12 est relativement négligeable. Autrement dit, en considérant que le réservoir-tampon 54 est rempli en excès du fait de l'apport en gaz par la source d'O₂ 4 et qu'une partie du gaz contenu dans le réservoir-tampon 54 s'échappe alors à l'ambient via la valve d'échappement 56, et par ailleurs la faible sélectivité pour le N₂ des fibres creuses 330 du module de séparation de CO₂ 33, un équilibre va s'établir dans le temps au cours duquel la concentration en N₂ dans les gaz circulant dans la ligne principale d'acheminement de gaz 53 et inhale par le patient, sera inferieure a 5%.

Dès lors, la concentration en O₂ du gaz inhalé par le patient P est elle-même supérieure à 95%, ce qui permet de corriger toute situation hypoxémique.

D'une façon générale, en associant une source d'O₂ 4, telle une bouteille d'O₂ comprimé 40, un système de purification 3 comprenant un (ou des) module de perméation CO₂ 33 pour éliminer le CO₂ présent dans les gaz expirés (i.e. mélange CO₂/O₂) et préférentiellement un système générateur de gaz frais 2 embarquant un système venturi, en particulier utilisant la puissance pneumatique de la source d'O₂ 4, il est possible, selon l'invention, d'augmenter l'autonomie de la source d'O₂ 4 afin de répondre à des situations nécessitant une administration d'oxygène pendant des périodes de temps jusqu'ici délicates à satisfaire.

## Revendications

1. Installation de fourniture (1) de gaz respiratoire à un utilisateur (P) comprenant :
- au moins une source de gaz (4) pour fournir un gaz respiratoire contenant de l'oxygène à un réservoir-tampon (54),
- une ligne principale d'acheminement de gaz (53) reliant fluidiquement le réservoir-tampon (54) à une interface respiratoire (51),
- une ligne de récupération de gaz (10) reliant fluidiquement l'interface respiratoire (51) à un système de purification de gaz (3) et
- une ligne de recyclage de gaz (11) reliant fluidiquement ledit système de purification de gaz (3) au réservoir-tampon (54),
**caractérisée en ce que** :
- le système de purification de gaz (3) comprend au moins un module de perméation membranaire (33), et
- elle comprend en outre un système générateur de gaz frais (2) comprenant un circuit de gaz interne (22) relié fluidiquement audit au moins un module de perméation membranaire (33) et à la source de gaz (4), le système générateur de gaz frais (2) comprenant une vanne de contrôle (23), un dispositif à orifice calibré (25) et un dispositif à venturi (26), agencés en série sur ledit circuit de gaz interne (22).

2. Installation selon la revendication 1, **caractérisée en ce qu'**un dispositif régulateur de précision (24) est agencé sur le circuit de gaz interne (22), entre la vanne de contrôle (23) et le dispositif à orifice calibré (25).

3. Installation selon la revendication1, **caractérisée en ce que** la vanne de contrôle (23) est une vanne à actionnement manuel.

4. Installation selon la revendication 1, **caractérisée en ce que** le dispositif à venturi (26) est raccordé fluidiquement à une sortie (25a) du dispositif à orifice calibré (25), ladite sortie (25a) du dispositif à orifice calibré (25) étant en communication fluidique avec un volume interne (260a) situé en aval d'un col de venturi (262) du dispositif à venturi (26).

5. Installation selon l'une des revendications 1 ou 2, **caractérisée en ce que** le diamètre de sortie, mesuré au niveau de la sortie (25a), du dispositif à orifice calibré (25) est inférieur à 500 µm.

6. Installation l'une des revendications 1 ou 4, **caractérisée en ce que** le dispositif à venturi (26) comprend un port de sortie (269) relié fluidiquement au module de perméation (33), via un conduit d'amenée de gaz frais (60).

7. Installation selon la revendication 1, **caractérisée en ce que** le ou chaque module de perméation (33) comprend des fibres membranaires creuses (330) agencées en parallèle les unes des autres.

8. Installation selon l'une des revendications 1 ou 7, **caractérisée en ce que** ledit au moins un module de perméation (33) comprend un port d'alimentation (33a) pour recevoir le gaz à purifier, un port de rétentat (33b) fournissant le gaz purifié, un port de perméat (33c) fournissant le gaz à éliminer et un port de gaz frais (33d) alimenté en gaz frais.

9. Installation selon la revendication 1, **caractérisée en ce que** ladite au moins une source de gaz (4) fournit un gaz respiratoire contenant plus de 80% d'oxygène, de préférence de l'oxygène pur.

10. Installation selon la revendication 1, **caractérisée en ce qu'**une valve d'inhalation unidirectionnelle (535) est agencée dans la ligne principale d'acheminement de gaz (53) et configurée pour autoriser la circulation de gaz respiratoire uniquement dans le sens allant du réservoir-tampon (54) vers l'interface respiratoire (51).

11. Installation selon la revendication 1, **caractérisée en ce que** l'interface respiratoire (51) comprend un masque respiratoire, en particulier un masque facial.

12. Installation selon la revendication 1, **caractérisée en ce que** le réservoir-tampon (54) comprend un ballon flexible ou un soufflet.

13. Installation selon les revendications 1 et 8, **caractérisée en ce que** chaque module de perméation (33) comprend une première chambre (333a) en communication fluidique avec le port d'alimentation (33a) et une deuxième chambre (333b) en communication fluidique avec le port retentât (33b).

14. Installation selon la revendication 13, **caractérisée en ce que** la première chambre (333a) et la deuxième chambre (333b) sont fluidiquement isolées l'une de l'autre, et séparées l'une de l'autre par une troisième chambre (333c) en communication fluidique avec le port de perméat (33c) et le port de gaz frais (33d).

15. Installation selon la revendication 1, **caractérisée en ce que** la vanne de contrôle (23), le dispositif à orifice calibré (25) et le dispositif à venturi (26) sont agencés dans un boitier commun (200).

## Patentansprüche

1. Eine Atemgasversorgungseinrichtung (1) für einen Benutzer (P), umfassend:
- mindestens eine Gasquelle (4) zur Versorgung eines Pufferbehälters (54) mit einem sauerstoffhaltigen Atemgas,
- eine Hauptgaszufuhrleitung (53), die den Pufferbehälter (54) fluidisch mit einer Atemschnittstelle (51) verbindet,
- eine Gasrückgewinnungsleitung (10), die die Atemschnittstelle (51) fluidisch mit einem Gasreinigungssystem (3) verbindet, und
- eine Gasrecyclingleitung (11), die das Gasreinigungssystem (3) fluidisch mit dem Pufferbehälter (54) verbindet,
**dadurch gekennzeichnet, dass**:
- das Gasreinigungssystem (3) mindestens ein Membranpermeationsmodul (33) umfasst, und
- sie ferner ein Frischgaserzeugungssystem (2) umfasst, das einen internen Gaskreislauf (22) aufweist, der fluidisch mit dem mindestens einen Membranpermeationsmodul (33) und der Gasquelle (4) verbunden ist, wobei das Frischgaserzeugungssystem (2) ein Steuerventil (23), eine kalibrierte Blendevorrichtung (25) und eine Venturi-Vorrichtung (26) umfasst, die in Reihe in dem internen Gaskreislauf (22) angeordnet sind.

2. Die Einrichtung nach Anspruch 1, **dadurch gekennzeichnet, dass** eine Präzisionsreglervorrichtung (24) am internen Gaskreislauf (22) zwischen dem Steuerventil (23) und der kalibrierten Blendevorrichtung (25) angeordnet ist.

3. Die Einrichtung nach Anspruch 1, **dadurch gekennzeichnet, dass** das Steuerventil (23) ein manuell betätigtes Ventil ist.

4. Die Einrichtung nach Anspruch 1, **dadurch gekennzeichnet, dass** die Venturi-Vorrichtung (26) fluidisch mit einem Auslass (25a) der kalibrierten Blendevorrichtung (25) verbunden ist, wobei der Auslass (25a) der kalibrierten Blendevorrichtung (25) in Fluidverbindung mit einem Innenvolumen (260a) steht, das sich stromabwärts eines Venturi-Halses (262) der Venturi-Vorrichtung (26) befindet.

5. Die Einrichtung nach einem der Ansprüche 1 oder 2, **dadurch gekennzeichnet, dass** der Auslassdurchmesser der kalibrierten Blendevorrichtung (25), gemessen am Auslass (25a), weniger als 500 µm beträgt.

6. Die Einrichtung nach einem der Ansprüche 1 oder 4, **dadurch gekennzeichnet, dass** die Venturi-Vorrichtung (26) einen Auslassanschluss (269) umfasst, der über einen Frischgaszufuhrkanal (60) fluidisch mit dem Permeationsmodul (33) verbunden ist.

7. Die Einrichtung nach Anspruch 1, **dadurch gekennzeichnet, dass** das oder jedes Permeationsmodul (33) Hohlfasermembranen (330) umfasst, die parallel zueinander angeordnet sind.

8. Die Einrichtung nach einem der Ansprüche 1 oder 7, **dadurch gekennzeichnet, dass** das mindestens eine Permeationsmodul (33) einen Zufuhranschluss (33a) zum Empfangen des zu reinigenden Gases, einen Retentatanschluss (33b) zum Zuführen des gereinigten Gases, einen Permeatanschluss (33c) zum Zuführen des zu entfernenden Gases und einen Frischgasanschluss (33d), der mit Frischgas versorgt wird, umfasst.

9. Die Einrichtung nach Anspruch 1, **dadurch gekennzeichnet, dass** die mindestens eine Gasquelle (4) ein Atemgas liefert, das mehr als 80% Sauerstoff, vorzugsweise reinen Sauerstoff, enthält.

10. Die Einrichtung nach Anspruch 1, **dadurch gekennzeichnet, dass** ein unidirektionales Einatemventil (535) in der Hauptgaszufuhrleitung (53) angeordnet und so konfiguriert ist, dass es die Zirkulation von Atemgas nur in der Richtung vom Pufferbehälter (54) zur Atemschnittstelle (51) zulässt.

11. Die Einrichtung nach Anspruch 1, **dadurch gekennzeichnet, dass** die Atemschnittstelle (51) eine Atemmaske, insbesondere eine Gesichtsmaske, umfasst.

12. Die Einrichtung nach Anspruch 1, **dadurch gekennzeichnet, dass** der Pufferbehälter (54) einen flexiblen Beutel oder einen Balg umfasst.

13. Die Einrichtung nach den Ansprüchen 1 und 8, **dadurch gekennzeichnet, dass** jedes Permeationsmodul (33) eine erste Kammer (333a) in Fluidverbindung mit dem Zufuhranschluss (33a) und eine zweite Kammer (333b) in Fluidverbindung mit dem Retentatanschluss (33b) umfasst.

14. Die Einrichtung nach Anspruch 13, **dadurch gekennzeichnet, dass** die erste Kammer (333a) und die zweite Kammer (333b) fluidisch voneinander isoliert und durch eine dritte Kammer (333c) voneinander getrennt sind, die in Fluidverbindung mit dem Permeatanschluss (33c) und dem Frischgasanschluss (33d) steht.

15. Die Einrichtung nach Anspruch 1, **dadurch gekennzeichnet, dass** das Steuerventil (23), die kalibrierte Blendevorrichtung (25) und die Venturi-Vorrichtung (26) in einem gemeinsamen Gehäuse (200) angeordnet sind.

## Claims

1. A respiratory gas supply installation (1) for a user (P) comprising:
- at least one gas source (4) for supplying a respiratory gas containing oxygen to a buffer tank (54),
- a main gas delivery line (53) fluidly connecting the buffer tank (54) to a respiratory interface (51),
- a gas recovery line (10) fluidly connecting the respiratory interface (51) to a gas purification system (3) and
- a gas recycling line (11) fluidly connecting said gas purification system (3) to the buffer tank (54),
**characterized in that**:
- the gas purification system (3) comprises at least one membrane permeation module (33), and
- it further comprises a fresh gas generation system (2) comprising an internal gas circuit (22) fluidly connected to said at least one membrane permeation module (33) and to the gas source (4), the fresh gas generation system (2) comprising a control valve (23), a calibrated orifice device (25) and a venturi device (26), arranged in series on said internal gas circuit (22).

2. The installation according to claim 1, **characterized in that** a precision regulator device (24) is arranged on the internal gas circuit (22), between the control valve (23) and the calibrated orifice device (25).

3. The installation according to claim 1, **characterized in that** the control valve (23) is a manually operated valve.

4. The installation according to claim 1, **characterized in that** the venturi device (26) is fluidly connected to an outlet (25a) of the calibrated orifice device (25), said outlet (25a) of the calibrated orifice device (25) being in fluid communication with an internal volume (260a) located downstream of a venturi throat (262) of the venturi device (26).

5. The installation according to one of claims 1 or 2, **characterized in that** the outlet diameter, measured at the outlet (25a), of the calibrated orifice device (25) is less than 500 µm.

6. The installation according to one of claims 1 or 4, **characterized in that** the venturi device (26) comprises an outlet port (269) fluidly connected to the permeation module (33), via a fresh gas supply duct (60).

7. The installation according to claim 1, **characterized in that** the or each permeation module (33) comprises hollow membrane fibers (330) arranged in parallel to one another.

8. The installation according to one of claims 1 or 7, **characterized in that** said at least one permeation module (33) comprises a supply port (33a) for receiving the gas to be purified, a retentate port (33b) for supplying the purified gas, a permeate port (33c) for supplying the gas to be eliminated and a fresh gas port (33d) supplied with fresh gas.

9. The installation according to claim 1, **characterized in that** said at least one gas source (4) supplies a respiratory gas containing more than 80% oxygen, preferably pure oxygen.

10. The installation according to claim 1, **characterized in that** a unidirectional inhalation valve (535) is arranged in the main gas delivery line (53) and configured to allow the circulation of respiratory gas only in the direction from the buffer tank (54) to the respiratory interface (51).

11. The installation according to claim 1, **characterized in that** the respiratory interface (51) comprises a respiratory mask, in particular a facial mask.

12. The installation according to claim 1, **characterized in that** the buffer tank (54) comprises a flexible bag or a bellows.

13. The installation according to claims 1 and 8, **characterized in that** each permeation module (33) comprises a first chamber (333a) in fluid communication with the supply port (33a) and a second chamber (333b) in fluid communication with the retentate port (33b).

14. The installation according to claim 13, **characterized in that** the first chamber (333a) and the second chamber (333b) are fluidly isolated from each other, and separated from each other by a third chamber (333c) in fluid communication with the permeate port (33c) and the fresh gas port (33d).

15. The installation according to claim 1, **characterized in that** the control valve (23), the calibrated orifice device (25) and the venturi device (26) are arranged in a common housing (200).
